# EUROPEAN PATENT SPECIFICATION

(11) **EP 3 198 003 B1**
(45) Date of publication and mention of the grant of the patent: **17.07.2024**
(21) Application number: 15845303.5
(22) Date of filing: 26.08.2015
(51) Int. Cl.: C12N 5/00

(54) **SEEDING AN ADHERENT CELL BIOREACTOR WITH NON-ADHERENT CELLS INCREASES SEEDING DENSITY LIMIT AND REDUCES REQUIRED EXPANSION TIME**
BEIMPFUNG EINES ADHÄRENTEN BIOREAKTORS MIT NICHT-ADHÄRENTEN ZELLEN ZUR ERHÖHUNG DER IMPFDICHTEGRENZE UND VERRINGERUNG DER ERFORDERLICHEN EXPANSIONSZEIT
ENSEMENCEMENT D'UN BIORÉACTEUR À CELLULES ADHÉRENTES AVEC DES CELLULES NON ADHÉRENTES AUGMENTANT LA LIMITE DE DENSITÉ D'ENSEMENCEMENT ET RÉDUISANT LE TEMPS D'EXPANSION REQUIS

(30) Priority: 26.09.2014 GB 201417042
(43) Date of publication of application: 02.08.2017
(73) Proprietor: Trizell Ltd., West Drayton, UB7 7PS (GB)
(72) Inventor: LESCH, Hanna, P., FI-70210 Kuopio (FI); KARHINEN, Minna, FI-70210 Kuopio (FI); HASSINEN, Minna, FI-70210 Kuopio (FI); SHAW, Robert, FI-70210 Kuopio (FI); PARKER, Nigel, Chinnor Oxfordshire OX39 4TW (GB); YLA-HERTTUALA, Seppo, FI-70210 Kuopio (FI); MALINEN, Joonas, FI-70210 Kuopio (FI); LIPPONEN,Eevi, FI-70210 Kuopio (FI)
(74) Representative: Cabinet Becker et Associés
(86) International application number: PCT/US2015/046927
(87) International publication number: WO 2016/048556

(56) References cited:
- EP-A1- 1 760 151
- WO-A1-02/074940
- WO-A1-2013/076309
- WO-A1-93/25071
- WO-A2-2011/112601
- US-A- 4 833 083
- US-A- 5 994 134
- US-A1- 2009 017 523
- US-A1- 2013 244 331
- US-A1- 2014 178 995
- ANONYMOUS: "Using Fixed-Bed Bioreactors in Adherent Cell Culture - Cell Culture Dish", 1 July 2014 (2014-07-01), XP055449026, Retrieved from the Internet <URL:http://cellculturedish.com/2014/07/using-fixed-bed-bioreactors-adherent-cell-culture/> [retrieved on 20180207]
- ANONYMOUS: "Mammalian Cells - HEK 293 INTRODUCTION", 1 January 2006 (2006-01-01), XP055449741, Retrieved from the Internet <URL:https://www.funakoshi.co.jp/data/datasheet/GTS/C503200.pdf> [retrieved on 20180209]
- ANONYMOUS: "Has anyone had any luck with increasing adherence/cell monolayer production with commercial products? Additives? If so, which ones? - ResearchGate", 19 November 2012 (2012-11-19), XP055449757, Retrieved from the Internet <URL:https://web.archive.org/web/20150929023152/https://www.researchgate.net/post/Has_anyone_had_any_luck_with_increasing_adherence_cell_monolayer_production_with_commercial_products_Additives_If_so_which_ones> [retrieved on 20180209]
- E N KNOWLES SHANE ET AL: "Linear scalability of virus production in the integrity iCELLis single-use fixed-bed bioreactors from bench to industrial scale", 4 December 2013 (2013-12-04), XP055449543, Retrieved from the Internet <URL:https://www.ncbi.nlm.nih.gov/pmc/articles/PMC3980293/pdf/1753-6561-7-S6-P60.pdf> [retrieved on 20180208]
- ALEXANDRE LENNAERTZ ET AL: "Viral vector production in the integrity TM iCELLis TM single-use fixed-bed bioreactor, from bench-scale to industrial scale", BMC PROCEEDINGS, BIOMED CENTRAL LTD, LONDON UK, vol. 7, no. Suppl 6, 4 December 2013 (2013-12-04), pages P59, XP021170358, ISSN: 1753-6561, DOI: 10.1186/1753-6561-7-S6-P59
- GENZEL YVONNE ET AL: "Vaccine production: upstream processing with adherent or suspension cell lines", 1 January 2014, ANIMAL CELL BIOTECHNOLOGY: METHODS AND PROTOCOLS IN: METHODS IN MOLECULAR BIOLOGY; ISSN; VOL. 1104; [METHODS IN MOLECULAR BIOLOGY; ISSN 1064-3745; VOL. 1104], HUMANA PRESS, US, PAGE(S) 371 - 393, ISBN: 978-1-62703-732-7, XP009503372
- RALF POERTNEROSCAR B J PLATAS BARRADAS ED - ED : PÖRTNER R: "Cultivation of Mammalian Cells in Fixed-Bed Reactors", 1 January 2007, ANIMAL CELL BIOTECHNOLOGY: METHODS AND PROTOCOLS. 2ND ED. (BOOK SERIES: METHODS IN BIOTECHNOLOGY), HUMANA PRESS, TOTOWA, NJ, USA, PAGE(S) 353 - 369, ISBN: 978-1-58829-660-3, XP009503387
- HANNA P. LESCH ET AL: "Process Development of Adenoviral Vector Production in Fixed Bed Bioreactor: From Bench to Commercial Scale", HUMAN GENE THERAPY, vol. 26, no. 8, 1 August 2015 (2015-08-01), & CONFERENCE ON CHANGING THE FACE OF MODERN MEDICINE - STEM CELLS AND GENE THERAPY; FLORENCE, ITALY; OCTOBER 18 -21, 2016, pages 560 - 571, XP055426642, ISSN: 1043-0342, DOI: 10.1089/hum.2015.081
- JORDAN M ET AL: "Transfection of adherent and suspended cells by calcium phosphate", MET, ACADEMIC PRESS, US, vol. 33, no. 2, 1 June 2004 (2004-06-01), pages 136 - 143, XP004505455, ISSN: 1046-2023, DOI: 10.1016/J.YMETH.2003.11.011
- R. TOM ET AL: "Transfection of Adherent HEK293-EBNA1 Cells in a Six-Well Plate with Branched PEI for Production of Recombinant Proteins", COLD SPRING HARBOR PROTOCOLS, vol. 2008, no. 4, 1 March 2008 (2008-03-01), XP055483084, DOI: 10.1101/pdb.prot4978
- RAMYA RAJENDRAN ET AL: "Assessment of packed bed bioreactor systems in the production of viral vaccines", AMB EXPRESS, 25 April 2014 (2014-04-25), Berlin/Heidelberg, pages 1 - 7, XP055483280, Retrieved from the Internet <URL:https://www.ncbi.nlm.nih.gov/pmc/articles/PMC4052670/pdf/s13568-014-0025-z.pdf> [retrieved on 20180612], DOI: 10.1186/s13568-014-0025-z
- JOHN GRAHAM: "Homogenization of Mammalian Cultured Cells", THE SCIENTIFIC WORLD JOURNAL, vol. 2, 1 January 2002 (2002-01-01), pages 1630 - 1633, XP055483355, DOI: 10.1100/tsw.2002.848
- CHANG T-Y ET AL: "A simple and efficient procedure for the rapid homogenization of cultured animal cells grown in monolayer", ANALYTICAL BIOCHEMISTRY, ELSEVIER, AMSTERDAM, NL, vol. 116, no. 2, 15 September 1981 (1981-09-15), pages 298 - 302, XP024826478, ISSN: 0003-2697, [retrieved on 19810915], DOI: 10.1016/0003-2697(81)90360-2

## Description

### Related Applications

This application claims priority from Great Britain patent application Serial No. 1417042.7 filed 25 Sept. 2014.

### Field

We have found a counter-intuitive way to improve the commercial-scale production of recombinant biological products in adherent-cell bioreactors. Our approach reduces the risk of cell culture contamination, increases total yield and reduces the delay between seeding and harvest, thus minimizing expression product degradation.

### Background

To date, there is only one approved gene therapy product, Glybera^{®}, which contains an adeno-associated virus for the treatment of a rare familial lipoprotein lipase deficiency (Salmon *et al.* 2014). Nevertheless, a broad spectrum of gene therapy applications show potential for future development and commercialization. Reaching patient doses of 10¹²-10¹³ viral particles (vp) for smaller-scale clinical trials has proved a difficult obstacle so far, and large scale manufacturing of viral vectors for commercial supply (i.e., ≥10¹⁶ vp) remains a challenge. The manufacturing challenge was recently reviewed by (Vellinga *et al.* 2014).

The range of producer cell lines is wide. Cells for *in vitro* culture come in two general types: cells adapted to live adhered to a solid substrate, and cells adapted to live in suspension in a liquid medium. Adherent cell lines include e.g., HEK293 (and derivatives like 293T and 293FT cells), CEF, MDBK, A549 and Vero. These have been used for the production of different biological products such as adenovirus, MVA, bovine herpes virus, AAV or influenza virus (Knowles et al. 2013).

Not all cells can be efficiently grown in suspension, and in addition, some cells in adherent conditions have shown higher specific vector productivity (Iyer *et al.* 1999). Therefore the efficient scaling up of the adherent production systems is required. The traditional way to culture adherent cells is in a flask, and these can vary in type (e.g. T-flasks, roller bottles, Cell Stacks, Hyper Stacks or Cell Factories). Upscaled production in flasks is limited by the production space required, it is impractical to handle and the multiple units make it difficult for monitor/ control culture conditions.

There are different kinds of carriers or substrates available for use in adherent-adapted cell culture bioreactors. For example bead-type micro-carriers (e.g., Cytodex, GE healthcare) float freely in suspension in media. Matrix type carriers (e.g., Fibra-cell disks, Eppendorf) consist of polyester fibers, optionally held within a cage (for example, of polypropylene) to immobilize the fibers. We here use the terms "carrier" and "substrate" to mean any solid-state material used to provide a surface onto which adherent cells may adhere in culture.

Bead-type microcarriers have been tried in bioreactors (Wu *et al.* 2002), the micro-carrier providing a substrate for adherent-cell growth while being dispersed in the cell culture medium. These systems, however, have not proven easy to handle, and do not ensure homogenous growth. Another limitation has been the expansion of large cell mass on the static vessels with limited scalability. They also need labor-consuming operations for their separation from the vector later in the process (Dormond *et al.* 2009).

Challenges in productivity are
1. Lack of controlled automatic production systems
2. Low single cell productivity which increases the cell number/volume
3. Lack of large scale bioreactor systems to support high productivity
4. Lack of disposable bioreactor systems to avoid demanding cleaning and sterilization steps in GMP
5. Challenges in functional feeding strategies (e.g. perfusion membrane blockage during the suspension cell culture)
6. Cell mass (cell density) is not enough to support high yield production (cell expansion step)
7. Low total yield per batch
8. Aggregation/precipitation during the downstream process

The use of packed-fiber bed adherent cell culture bioreactors have provided optional controlled, perfusable system with low shear stress (Meuwly *et al.* 2007). The iCELLis^{®} bioreactor (commercially available from Artelis S.A (Paris France) / Advanced Technology Materials Inc. (Brussels, Belgium) / Pall Corporation (Fall River, Massachusetts) represents a fixed-bed bioreactor developed for scalable adherent VERvert Origin (VERO) cell cultures. iCELLis is a single-use compact fixed-bed bioreactor with a carrier made of polyester fibers. The commercial iCELLis system varies between 0.53 and 500 m², having two different compaction densities of the carriers (Knowles et al. 2013). Such bioreactors have been used for different kinds of adherent cell process systems. These include the use of different cell lines, such as HEK293, CEF, MDBK, A549 and Vero, and for the production of different biological products, such as adenovirus, MVA, bovine herpes virus, AAV or influenza virus (Knowles et al. 2013) (Lennaertz *et al.* 2013) .

Even though large capacity adherent cell culture systems provide a theoretically-attractive production system, the expansion of the cell mass prior to initial loading for a large scale adherent culture bioreactor can become a major obstacle during the process. It has been calculated that 15 times 1700cm² roller bottles or 30 times 850 cm² roller bottles was needed for the loading of Vero cells into an adherent culture bioreactor providing 500m² of adherent cell culture area (Knowles et al. 2013). However, the seeding density is cell line-dependent and needs to be developed for each individual process. When they were using 293T cells, the loading density was 25 times higher (80 000 cells/cm²) (Lennaertz *et al.* 2013) which means that to a inoculate an adherent culture bioreactor providing 500m² of adherent cell culture area, they would have required 4 × 10¹¹ cells expanded approximately in 1500-2000 roller bottles. We used less cells for inoculation and calculate that the production in one 500 m² fixed bed bioreactor system the culture area is equal to 6000 times 850cm² roller bottles when about 500 roller bottles are needed for the expansion of adequate cell mass (somewhat depends on the cell density inoculated). Standard calculations using the same cell density as a standard 175cm² flask for the inoculation phase show that the expansion of the cell mass for a 500m² fixed bed bioreactor would require around 2000 times 175 cm² T-flasks.

Furthermore, large numbers of flasks are expensive to purchase and set up. Transferring adherent cells from large numbers of flasks into a bioreactor can be difficult because an operator must treat each flask individually with e.g. trypsin to remove the adherent cells from the flask. The larger the number of flasks, the greater the risk that one flask will become contaminated. When the contents of numerous T-flasks are combined into a single bioreactor, that contamination could destroy the entire production run. Thus, using a large number of flasks to expand adherent cells has been considered infeasible in making commercially-acceptable quality biologic products.

Currently both adherent and suspension methods for viral vector production are commonly used (Kamen & Henry 2004; Segura *et al.* 2007). We have previously also produced different viral vectors using a variety of suspension adapted cell culture systems (e.g., the Cultibag^{®} brand polypropylene bag suspension bioreactor and the Mobius CellReady^{®} stirred tank suspension cell culture bioreactor using suspension adapted cells, or flasks using suspension-adapted cell culture systems. A particular recombinant adenovirus type 5 product has low productivity in suspension cells (<15 000 vp/cell) whereas in traditional adherent production system the productivity is high (>50000 vp/cell). upscaling of an adherent system has, so far, not been possible, in an economically-viable way. For example, an adherent culture bioreactor providing 500m² of adherent cell culture area on polyester fiber carrier now provides an upscaling possibility but the use of such an adherent system is not possible, due to the need for hundreds of Triple flasks or roller bottles for the large-scale seeding requirements. Cell expansion, as part of the adherent system, is therefore a major obstacle.

While such challenges inhibit adherent systems' adoption for commercial scale manufacturing, if these challenges could be overcome, an adherent system would offer a critical advantage: adherent cells produce more viral particles than do suspension cells. Thus, even though several suspension approaches are available for viral vector production, an adherent cell line could provide a crucial advantage in commercial-scale manufacturing because the productivity of viral vector in adherent cells can be much higher than in suspension. Thus, while the prior art for many reasons discourages the use of Foetal Bovine Serum, we have found that the addition of Foetal Bovine Serum can increase viral vector productivity significantly. This phenomenon appears to be due to the lipid content of Foetal Bovine Serum, which lipids may be a key component during viral vector (especially enveloped viral vector) production to increase the yield and the stability of the resulting vector. In addition, certain cell lines cannot be grown in suspension mode, so being able to productively use these lines in commercial-scale manufacture requires finding a way to adapt adherent culture to commercial-scale manufacture. US4833083 discusses packed bed bioreactors. EP1760151 discusses adenovirus compositions obtainable by a production and purification method. WO02074940 discusses methods of extracting viruses from cell culture. WO2011112601 discusses continuous flow bioreactors for magnetically stabilized three-dimensional tissue culture. Graham et al. (2002) The Scientific World Journal, 2: 1630-1633 discusses the homogenization of mammalian cultured cells. Chang et al. (1981) Anal. Biochem., 116(2): 298-302 discusses a simple and efficient procedure for the rapid homogenization of cultured animal cells grown in monolayer. Rajendran et al. (2014) AMB Express, 4: 25 discusses an assessment of packed bed bioreactor systems in the production of viral vaccines. Jordan et al. (2004) Methods, 33(2): 136-43 discusses transfection of adherent and suspended cells by calcium phosphate. Lesch et al. (2015) Hum Gene Ther, 26(8): 560-71 discusses the process development of adenoviral vector production in a fixed bed bioreactor. Cellculturedish.com published an article in 2018 directed to using fixed-bed bioreactors in adherent cell culture.

Accordingly, when using such bioreactors and cell growth in adherent mode, there exists a need in the art for an effective means of cell expansion to obtain the requisite initial cell mass for initial loading of the bioreactor.

### Summary

The art teaches to seed adherent-cell bioreactors with cells adapted to adherent culture. We tested the exact opposite approach, and tried seeding an adherent-cell bioreactor with suspension-adapted cells. We surprisingly found that not only do suspension-adapted cells adhere to the substrate in the adherent bioreactor, but those suspension-adapted cells thrive when cultured in adherent mode, and using suspension-adapted cells enables one to greatly reduce the amount of cells required for seeding, and avoids clogging the substrate or carrier. The present invention is thus based, at least in part, on the finding that suspension-adapted cells (expanded in suspension culture) can be successfully inoculated into an adherent-culture bioreactor, and subsequently grown in adherent mode. This overcomes the problem of there currently existing no commercially-viable means of expanding cells, so as to reach the required cell mass for loading of commercial-scale bioreactors which make use of adherent cells.

This approach runs contrary to common practice, where it is thought that cells should be expanded in adherent mode prior to their use in adherent production systems. Indeed, using non-adhering suspension cells for expansion is against the express instructions and recommendations of at least one adherent-culture bioreactor manufacturer (Advanced Technology Materials Inc. (Brussels, Belgium) / Pall Corporation (Fall river, Massachusetts)).

According to a first aspect of the invention, there is provided a method for the production of a recombinant biological product, comprising: (a) obtaining cells that have been expanded in suspension mode; and (c) inoculating the cells into a bioreactor having a packed-fiber carrier providing a surface area for adherent cell culture; and then (d) exposing the cells to a factor promoting adherence selected from the group consisting of: fetal bovine serum, collagen, fibronectin, laminin, calcium ions, proteoglycans and ECM polysaccharides, in an amount effective to promote adherence, whereby the cells adhere to said packed-fiber carrier in said bioreactor

According to a second aspect of the invention, there is provided the use of suspension-adapted cells in a method for the production of a recombinant biological product; wherein said suspension-adapted cells have been expanded in a suspension cell culture; and wherein the method comprises inoculating the expanded cells obtained from the suspension culture into a bioreactor having a packed-fiber carrier, and then exposing the cells to a factor promoting adherence selected from the group consisting of fetal bovine serum, collagen, fibronectin, laminin, calcium ions, proteoglycans and ECM polysaccharides, in an amount effective to promote adherence, whereby after inoculating the cells, the cells adhere to the packed-fiber carrier in the bioreactor and the expanded cells are cultured in adherent mode to produce the recombinant biological product.

Our counter-intuitive approach provides a new way to greatly reduce the need for down-stream processing of expression products which require host cell lysis for isolation and purification, particularly products of suspension-adapted producer cell lines. For products requiring lysis of the producer cells, a fixed-bed bioreactor functions as a filter or "pre-clarification" when a lot of cell debris remains attached to or filtered by the fixed bed adherent cell culture substrate, and the burden for downstream is thus remarkably less. We observed that the harvested material is pretty clear compared to material harvested from suspension type bioreactors after cell lysis. Cellular DNA digestion outside the bioreactor prevented the aggregation of the virus since no aggregation was detected. We saw the same phenomenon if Benzonase treatment was performed before and after the producer cell chemical lysis inside the bioreactor followed by addition of high salt containing buffer.

### Brief Description of the Drawings

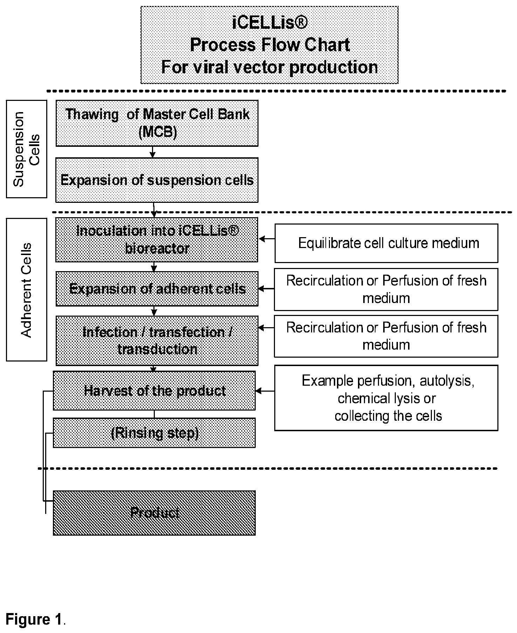

**Figure** 1 is a process flow chart showing typical steps involved in using our process to produce gene therapy viral vector in an iCELLis^{™} brand adherent-culture bioreactor.
Figure 2 shows typical results from our process using an iCELLis Nano^{™} brand adherent-culture bioreactor after expanding cells in suspension mode, inoculating the suspension-adapted cells into an iCELLis Nano^{™} brand adherent-culture bioreactor and then growing the cells in adherent mode. a) HEK293 cell growth curves. b) 293T cell growth curve. Glucose consumption and lactate accumulation correlated with cell growth.
Figure 3 is a fluorescent microscopy picture of 293T cells 24h after calcium phosphate transfection for the production of AAV2 expressing green florescent protein (GFP). 293T cells were expanded in suspension, inoculated into the iCELLis Nano^{™} brand 4m² bioreactor and grown in adherence mode during the AAV expression.
Figure 4. Testing the effect of different Benzonase concentration and inoculation time for the DNA clearance of harvested adenovirus material.
Figure 5. Turbidity vs time for various virus harvest samples.

### Detailed Description

### 1. Producer Cell line

Further according to said first and second aspects, the cells for use in the invention may comprise those of any suitable producer cell line, depending on the desired recombinant biological product, for example any eukaryotic producer cell line (e.g. immortalised mammalian cell line, or insect cell line). Preferably, this cell line is selected from the group consisting of HEK293, 239T, or any HEK293 related cell line HT-1080, HepG2, A549, CHO, BHK, Sf9, Sf21, NCL, UR, N52.E6, BTI-Tn 5 B 1-4, COS, NIH/3T3, Vero, NSO, NCST11, PerC6 or Her cells; or combinations or modifications of thereof. HEK293 and 293T cells are, however, more preferable; and furthermore are especially preferred when the recombinant biological product is a viral vector.

### 2. Adherent bioreactor type

Further according to said first and second aspects, it is preferable that the bioreactor comprises at least one, more preferably a plurality of carriers onto which the expanded cells are intended to adhere, which may be either floating or fixed in the bioreactor. Preferably, said carriers can be made, for example, using polyethylene terephthalate, polystyrene, polyester, polypropylene, DEAE-dextran, collagen, glass, alginate or acrylamide. Suitable bioreactors which may be used include those containing bead-type micro-carriers (e.g., Cytodex^{®} brand beads, commercially available from GE Healthcare Inc. division of General Electric Corp.) and matrix type carriers (e.g., Fibra-cell^{™} brand disks, commercially available from Eppendorf Corp.). It is especially preferred that the bioreactor uses a polyester fiber carrier such as that used in the iCELLis^{®} nano or iCELLis^{®} 500 bioreactors, (commercially available from Advanced Technology Materials Inc. (Brussels, Belgium) and Pall corporation (Fall River, Massachusetts).

### 3. Suspension expansion methods

Further according to said first and second aspects, it is preferable that the suspension culture comprises either the use of a flask-type expansion method, or a further bioreactor in which the cells are expanded in non-adherent (suspension) mode. The flask-type suspension expansion method can be any available in the art, using any type of cell culture flask suitable for steady or mixed/shaked suspension cell expansion using for example a T-flasks, roller bottle, spinner flask or shaker flask; or combinations thereof. Similarly, the further bioreactor can be any available in the art in which cells are expanded in suspension, for example a any batch, fed-batch or continuous bioreactor types, single-use or multiuse, stirred tank or wave type bioreactor, perfusion or recirculation type bioreactor; or combinations thereof. The exact method of suspension culture is not critical to the invention. The limiting factor for growth in any suspension culture is the concentration of cells in the medium (and not the surface area, as with adherent cell cultures). Accordingly, the use of any suitable suspension culture, when performed by the skilled person, will enable the reaching of a higher cell mass than an adherent culture of a similar scale, which can then be used for loading of a bioreactor (and subsequent culturing in adherent mode). Suitable mediums for cell expansion in suspension culture will also be apparent to the skilled person. For example, for the growth of HEK293 cells, culture CD293, BHK, Ex-cell GTM3, Freestyle or Hyclone SFM293 culture media may be used in a preferred embodiment, by way of example and not limitation, Ex-Cell medium (available from Sigma Aldrich Co. LLC) may be used in either flask-type or bioreactor-based expansion methods (detailed above), possibly containing L-glutamine (for example at a concentration of 6mM); such medium lacks calcium and other components required for cell attachment, thereby promoting suspension adaptation of the cells. Penicillin/streptomycin, while not preferred for cGMP manufacturing, may be included, for example at a concentration of about 50µg/ml, to avoid contamination. Preferably, the suspension-adapted cells in (a), of the first and second aspects of the invention, are expanded in a growth medium which is substantially free of (that is, comprising no more than trace levels of) calcium ions, foetal bovine serum (FBS), fibronectin, collagen, laminin, or proteoglycans or non-proteoglycan polysaccharides of the extracellular matrix which would support the anchorage of cells.

### 4. Inoculation

Further according to said first and second aspects, it is especially preferred that the medium containing the expanded cells during/after the inoculation in (b) comprises factors which promote cell adherence, preferably to the carriers of the bioreactor (see paragraph below), for example FBS, fibronectin, collagen, laminin, calcium ions, or proteoglycans or non-proteoglycan polysaccharides of the extracellular matrix; or combinations thereof. FBS (or another factor for promoting adherence) can be added to the culture medium just before, during or after the inoculation of the suspension cells into bioreactor. By way of example and not limitation, Dulbecco's modification of Eagle's (DMEM) medium (as is known to the skilled person) may be used, containing FBS (for example between 2 and 20% by weight of the medium, preferably between 5 and 15%, e.g. 10%), L-glutamine (for example at a concentration of 2mM) Inoculation of the bioreactor with an effective quantity of suspension-expanded cells will be performed, with said quantity varying depending on the bioreactor to be used. By way of example and not limitation, when using an iCELLis^{®} nano 4m² bioreactor, between 1×10⁸ and 1×10⁹, preferably between 3×10⁸ and 7×10⁸, more preferably between 4×10⁸ and 6×10⁸ cells, e.g. 5.2×10⁸ cells obtained from the expansion step may be used or equivalent cells density if any other size of the adherent bioreactor is used. Said cells may be centrifuged to obtain a cell pellet, and suspended in an appropriate volume of growth medium (for example, 100-300ml, e.g. 200ml) as defined below, before inoculation into e,g. the iCELLis^{®} nano adherent bioreactor. It is also possible to add cells into the bioreactor directly from suspension culture as defined below. For example, using Excell medium during cell expansion, this can be connected directly into the bioreactor containing, for example, DMEM with or without FBS. It was shown that the small quantity of suspension medium will not affect the cell anchorage. Accordingly, the cells in suspension culture may be transferred to the adherent culture bioreactor by any suitable means, including direct transfer, or by indirect transfer. For example, the cells may be transferred directly from the suspension reactor vessel e.g. a Cultibag^{™} polypropylene suspensioin culture bag to an adherent bioreactor, This is a major practical advance compared to the current flask type expansion systems where several handling steps like cells detached from the flasks (or carriers), are needed, which handling steps increase both cost and risk of contamination. This further proves the suitability of the manufacturing method for commercial production.

### 5. Infection/transfection/transduction conditions

Depending on the product in question, the stage of the method at which the recombinant biological product is introduced into the producer cells, and in what form, will vary. For example, a recombinant protein may be introduced into the producer cell as a coding polynucleotide (using standard methods available in the art), which will be performed prior to expansion and culturing. In contrast, a viral vector may be introduced through the infection of already-expanded cells, inside the bioreactor. Stable cell lines contains product related elements and the production can be continues or some promoter activation /regulation may be used. Necessary variations in the method, of the kind detailed above, will be apparent to the skilled person, depending on the recombinant biological product to be produced.

Viral vectors, and their production by transduction, transfection or infection, of expanded cells inside the bioreactor are, however, especially preferred. Transduction may be performed by introducing foreign DNA into a cell via viral vector or virus in order to produce the recombinant biological product. Transfection requires the use of one or more plasmids encoding the elements required for viral vector production, and may be performed by methods conventional in the art, including transfection, electroporation, cell squeezing, sono poration (non-chemical methods) or transfection reagents (chemical based methods), such as calcium phosphate or cationic lipid such as PEI. Infection is the preferred method of viral vector production, and can be achieved by a number of methods available in the art. We have used a multiplicity of infection (MOI) of up to about 400 virus particles/cell, but another suitable MOI may be used. By way of example and not limitation, for an adherent bioreactor such as the iCELLis^{®} nano, the viral vector drug product can be added to growth medium (for example, 500-1500ml, preferably 600-1200ml, e.g. 800ml growth medium) and circulated in place of spent medium or using perfusion. By way of further example and not limitation, cells can be infected/transfected/transduced when in their exponential growth phase in the bioreactor, for example for HEK293 or HEK293 related cells (e.g.293T) at a cell density of 100,000-300,000 cells/cm², preferably 150,000-250,000 cells/cm², more preferably 170,000-230,000 cells/cm²; although higher cell densities (for example >300,000 cells/cm²) may also be targeted for infection.

### 6. The product

According to said first and second aspects, the recombinant biological product can be any that is known in the art to be suitable for production in a bioreactor which uses adherent cells. Said product need not be limited to a viral vector specifically, given that the invention lies in the successful inoculation of suspension-expanded cells into, and their subsequent adherence onto, the bioreactor. Further according to said first and second aspects, preferably the recombinant biological product is a viral vector, viral-like particle, virus, recombinant protein, immunoglobulin, or viral-based vaccine.

Further according to said first and second aspects, when the recombinant biological product is a viral vector, preferably it is selected from the group consisting of adenovirus, adeno-associated virus, lentivirus, retrovirus, herpes simplex virus, vaccinia virus, measels virus, baculovirus and pox virus. Particularly preferred are adenovirus, lentivirus or AAV. Suitable methods for introducing the different types of viral vector into producer cells will be apparent to the skilled person, and include, for example, transfecting producer cells with plasmid(s) for lentiviral vector or AAV production. More preferably, the recombinant viral vector is an adenovirus vector (either replication competent or deficient). Production of replication-deficient adenovirus serotype 5 vectors, AAV or lentivirus is, however, especially preferred.

Further according to said first and second aspects, when the recombinant biological product is a viral vector, the vector will comprise a therapeutic transgene, preferably a therapeutic transgene or any therapeutic genetic element, such as siRNA, miRNA, shRNA. For example, the transgene can code for e.g., a vascular endothelial growth factor (VEGF) such as e.g., short-form VEGF-D3; a vascular growth inhibitor (e.g., endostatin, angiostatin); thymidine kinase (or another polypeptide with similar enzymatic activity); an interferon (e.g., human interferon alpha-2b) or another polypeptide with similar immune-modulating activity; an ATP-binding cassette (ABC) transport protein such as ABC1 member 4 (a/k/a ABCA4) or an ALD protein, e.g., the ABCD-1 protein; myosin VIIA; cyclooxygenase-2; a PGF2-alpha receptor; dopamine; the human hemoglobin subunit beta protein; or a monoclonal antibody subunit(s). We prefer that the transgene have about ≥95% identity to the cDNA sequence of the naturally-occurring polypeptide.

Further according to said first and second aspects, the combination of the replication-deficient adenovirus serotype 5 vector with a human interferon alpha-2b transgene is especially preferred. Said vector may be constructed with the human gene in an expression cassette which replaces the E1a, E1b and pIX regions at the 5' end of the adenovirus genome, as is known in the art. Such vector construction can be performed by the skilled person, using standard DNA manipulation techniques.

Alternatively, the vector may be a lentiviral vector, for example a lentiviral vector carrying a transgene coding for e.g., a VEGF, endostatin, angiostatin, thymidine kinase ABCA4, ABCD-1, myosin VIIA, cyclooxygenase-2, PGF2-alpha receptor, dopamine, the human hemoglobin subunit beta protein or a monoclonal antibody subunit(s).

Further according to said first and second aspects, it is especially preferred that the vector described in the preceding paragraph is produced by the method of the invention, wherein HEK293 or 293T cells (expanded in suspension, by no particular means) are inoculated into an iCELLis^{®} nano 4m² bioreactor using medium comprising preferably between 2×10⁸ and 8×10⁸ cells, e.g. 5.2×10⁸ cells or iCELLis^{®} 500 100m² between 5 × 10⁹ and 2×10¹⁰, e.g. 1×10¹⁰ cells when the seeding density can vary from 5000 to 20 000 cells per cm². The seeded cells may optionally then be expanded in the bioreactor. Said seeded cells are then infected/transfected or transduced inside the bioreactor. One may use e.g. the above viral vector at an MOI of greater than 200 virus particles/cell, preferably when at a cell density of 170,000-230,000 cells/cm². Alternatively, one may use a lower MOI; we prefer an MOI of not more than about 10 virus particles/cell. Virus production inside the bioreactor is performed for at least 24, preferably at least 48 hours, prior to harvesting of the virus.

### 7. Harvesting of the Product

The recombinant product may be obtained from the cultured cells in any convenient way. Some products are released into the cell culture medium so harvest of the product is performed by harvesting the medium. Medium can be harvested by perfusion (continuous collection of the product). Harvesting of the medium can be performed also by draining the medium out from the bioreactor at the end of the production or medium can be collected several times during the trial and replacing fresh medium into the bioreactor. For example lentivirus is budded into the cell culture medium and harvesting of the medium after the transfection can be performed for few days.

Some products are produced inside the cell and not released to the medium so the release of the product from the cells is needed. In addition, some viruses are lytic viruses, such as adenovirus, and their formation takes place inside the cells. Cell lysis may be carried out by any conventional means. Preferably, lysis is carried out by use of a detergent, e.g. Tween, preferably Tween 20 or Triton-X, or lysis can be happen as autolysis if e.g. lytic viruses are being produced. Finally many viruses can lyse the cell but it can be preferred to harvest the product that is still inside the cells. The traditional method is to collect the cells and release the product by freeze-and-thaw. Cells can by harvested from the flasks or bioreactor by cell dissociation where adherent cells are removed from culture surface by chemicals. Trypsin or EDTA or any derivative or any appropriate reagent can be used for cell removal. Alternative lysis can be performed by hypotonic solution, such us using water.

Additionally cells can be lysed inside the bioreactor. Chemical lysis using for example Triton-X or Tween break the cell membrane and release the product into the medium/buffer. The product can be harvested by collecting the lyzed material. Also autolysis can be used as a release option where virus production is prolonged and cells are lyzed naturally as a consequence of the infection. Typically autolysis takes few days longer that if harvesting the product from the cells. Purification of the released recombinant product can be carried out using conventional purification methods.

Lysis of the cells releases the product into the supernatant, but it also releases contaminants, such as intracellular proteins or genomic nucleic acids. High viral particle concentration and/or high concentration of contaminating components have been shown to cause viral aggregation which can lead finally to low total yield or even rejection of the batch. Interestingly, we showed for the first time the efficient cell lysis inside the fixed-bed bioreactor using lysis buffer which in this case was Tween20 based lysis buffer. We noticed that the harvested material was remarkable more clear compared to lysing the suspension cells or cells harvested as intact cells and lyzed by freeze-and-thaw. Fixed-bed functioned as a "pre-filter" keeping a lot of cell debris inside the carrier bed when the primary harvest was pretty clear. This was a remarkable improvement because the loading material for purification was much pure.

It was noticed that not all the product may be harvested by draining the bioreactor empty. Rinsing of the bioreactor may recover some product still attached on the cell debris and carrier fibres. Rinsing can be done by cell culture medium or any other suitable buffer. Rising step is unique way of recovering more virus particles (or any other biological products) and is not applicable for example in suspension bioreactors in this context.

### 8. DNA digestion and prevention of precipitation

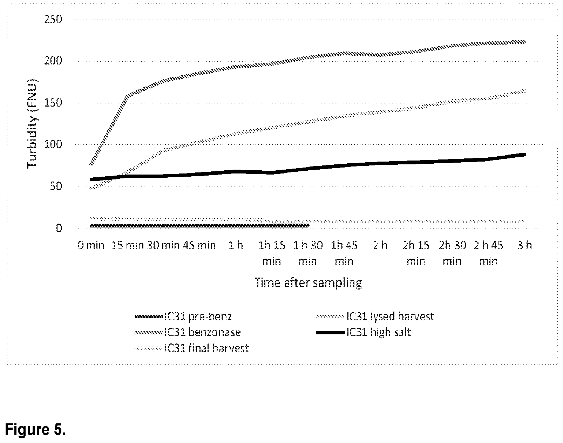

Removal of residual DNA from the clinical product is required by the authorities. In addition, the presence of contaminants, such as cellular nucleic acids or proteins virus may accelerate the aggregation of the product. The aggregation of the virus or any precipitation is a known problem during the processing of the product and may end up into remarkable loss of the product or even a rejection of the total batch. We were able to digest the genomic DNA and avoid visible aggregation/precipitation of the adenovirus by digesting cellular genomic DNA using DNAse endonuclease, in this cased called Benzonase when the digestion was performed outside the bioreactor, as a part of downstream processing of the product. On the other hand, if small amount of Benzonase was added before the lysis and more enzyme was added to the bioreactor after the lysis, virus aggregation was prevented when high salt was added after the Benzonase digestion into the supernatant (Figure 5).

### 9. Conclusion

The methods of the invention produce cells and cell lines of high commercial value. For example, a suspension-adapted cell capable of being grown in adherent culture conditions will be of use in the production of recombinant products. The cells may be obtained by the methods disclosed herein, however, alternative methods may be apparent to the skilled person. The invention outlined herein reveals, for the first time, that suspension-adapted cells can be made to revert to an adherent character. Now that this is appreciated the skilled person will recognise that there may be many ways to achieve re-adherence. Accordingly, the cells produced by the methods of the invention are not restricted to the particular method of production, but must share the same characteristics as the cells described herein; i.e. they must be adherent cells adapted for growth in suspension culture conditions and subsequently conditioned for re-adherence.

We now describe our invention by the following non-limiting Examples.

### Examples

### 1. Producer Cell line

In these experiments two commonly used cells lines, HEK293 and 293T cells were used as case examples. Adherent HEK293 or 293T cell lines were cultivated at 37°C and 5% CO₂ in cell culture flasks using Dulbecco's Modified Eagle Medium (Sigma-Aldrich) competed with 10% fetal bovine serum (FBS, Life Technologies), 2mM L-glutamine (Invitrogen). Optionally, one can add 50 µg/ml/50 IU/ml penicillin/streptomycin (Invitrogen). In suspension HEK293 or 293T cells were grown using EXCELL 293 serum-free medium (Sigma-Aldrich) containing 6 mM L-glutamine and 50 µg/ml penicillin/streptomycin. Other mediums, such as CD293 (Invitrogen), BHK (Merck Millipore), Ex-Cell GTM3 (Sigma-Aldrich), Freestyle (Invitrogen) and SFM293 (HyClone) have also been used for the growth of these cells in suspension. In the bioreactor, adherent cells were cultured using DMEM containing 10% FBS, 2mM L-glutamine. We in fact used 50µg/ml penicillin/streptomycin for this Experiment, but we prefer to avoid using it in actual commercial-scale manufacturing if possible.

### 2. Adherent bioreactor type

In these examples, an iCELLis^{®} Nano bioreactor (Pall Life Science) with Advance or MyControl (Applicon Biotechnology) control units and BioXpert software were used. The iCELLis^{®} Nano bioreactors 0.8m2 (2 cm bed height, carrier compaction 144 g/L), 2.67m² (10 cm bed height, carrier compaction 96 g/L) and 4m² (10 cm bed height, carrier compaction144 g/L) were tested. Calibrated probes installed into the bioreactor were sterilized by autoclaving 45 min. 121°C. Cells were inoculated using a density between 6000 and 13000 cells/cm² and two feeding strategies were used, either fed-batch mode by re-circulation medium or perfusion. The pH set point was 7.2, and was controlled by head space CO₂ or 7.5 % Sodium bicarbonate (Life Technologies). Dissolved Oxygen (DO) set point was 50%, controlled by stirring and head space air or O₂ flow. Cell density, pH and glucose/lactate were monitored daily. Glucose and Lactate were analyzed by Reflectometer (MerckMillipore) and pH was off-line measured by SevenGo Pro SG8 (Mettler Toledo). Carrier fiber strips were taken from the top of the bioreactor by using sterile tweezers, vortexed in 1 ml Lysis solution A (Fisher Scientific) for 5-10 min, and nuclei were counted by using hemocytometry and microscopic inspection. The biomass was monitored using an ABER Biomass monitor (Applicon Biotechnology), and FuturaLite software.

Cell density, pH, glucose and lactate were analyzed daily from iCELLis Nano. Carriers were taken from the bioreactor by using sterile tweezers. Three carriers were vortexed in 1 ml Lysis solution A for 5-10 min and nuclei were counted by using hemocytometer and microscope. One carrier was immersed in 1 ml crystal violet and rinsed 3x1 ml PBS. The attached cells were observed under microscope. One carrier was immersed in 1 ml Trypan Blue and rinsed 3x1 ml PBS. Dead cells were observed under microscope. About 5-8 ml medium was taken from the bioreactor for pH measurement and also glucose and lactate were analysed from a sample diluted 1:100 to PBS.

Also, the iCELLis^{®} 500 control system (Pall Life Science) together with Futura Scada Software was used for large scale bioreactor runs. The iCELLis^{®} 500 bioreactor was provided with a cassette housing the PET fibre bed of either a 500m² (10 cm bed height, carrier compaction144 g/L) or 100m² (2 cm bed height, carrier compaction 144 g/L) surface area for cell growth. A 500m² bioreactor was used to study the cell distribution in a bioreactor. A 100m² bioreactor was used in the first virus production experiment. Culture parameters were the same as in iCELLis^{®} Nano experiments. The medium was sampled daily and glucose, lactate and pH were measured off-line. BioWelder and BioSealer (Sartorius Stedim Biotech S.A.) were used for forming sterile tubing connections and seals during the production. The total working volume in the iCELLis^{®} 500 100m² was 65L. At the time of harvest, the carriers were treated in-situ with 1 % Lysis buffer in growth medium for 2 h at 37°C, continuously stirring the medium in the bioreactor fixed bed. Virus containing medium was drained out and carriers were rinsed with one working volume to recover more viruses from the fixed-bed.

### 3. Suspension expansion methods

There are different kinds of suspension expansion methods available. In suspension we cultured HEK293 or 293T cells either in roller bottles (Corning Incorporated Life Sciences), shaker flasks (Corning Incorporated Life Sciences), spinner flasks (Cornin Incorporated Life Science), in BIOSTAT^{®} CultiBag RM perfusion system (Sartorius Stedim Biotech S.A.) or in Wave Bioreactor (GE Healthcare) in a 0.5×10⁶ cells/ml seeding density (twice per week). Ex-Cell medium by Sigma-Aldrich Co. LLC was used in these examples. It is developed to support suspension cell growth and lacks e.g. calcium and other components for cell attachment. No attachment of HEK293 nor 293T cells into bottle falls was observed. Cells were passaged in suspension until used to inoculate iCELLi^{®} nano bioreactor.

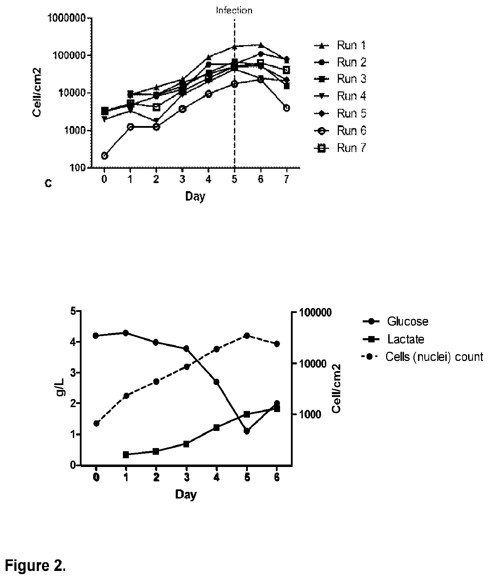

The first experiment to study the suspension expansion and attachment of the cells into the bioreactor was carried out without any virus production (see also inoculation studies 4. Inoculation). An iCELLis^{®} Nano 10 cm bed height bioreactor (4m²), was inoculated with suspension HEK293 cells expanded in roller bottles using seeding density of 13 000 cells/cm² and cells were cultivated for five days. Carriers from the top of the bioreactor were sampled daily and a nuclei count wasperformed (figure 2a). At the end of the run, the bioreactor was broken open and carriers were sampled from the bottom, middle and the top of the fixed fiber-bed. The average of the cell density was 172 600 cells/cm² but there were many more cells on the bottom carriers than on the top ones. Thus, it was concluded that cells were well grown in adherence but unevenly distributed within the fixed-bed (Table 1). This phenomenon explains why we were not able to count equivalent number of cells from the top carriers, than were inoculated into the bioreactor.

The first large-scale iCELLis^{®} 500 run focused only on suspension expansion in a large scale and adherent cell growth testing, but this time using a lower seeding density of 6000 cells/cm2, in order to explore the possibility of an "overgrowth" of the cells on the bottom carriers. Cells were entrapped and attached into the fixed fiber-bed, and then grown adherency for five days. The bioreactor was drained and opened to allow the fiber carriers to be sampled from the bottom, middle and top layers. Because of the larger diameter of this bioreactor's fixed bed, carriers were sampled from five different radial locations within each layer, to investigate variability and stratification. The results showed that there was not a homogenous distribution of the cells in iCELLis^{®} 500, but the differences were not that dramatic (Table 1). The average density was 103 600 cells/cm2. Cells were better distributed in the larger scale iCELLis^{®} 500, allowing the possibility to increase the cell density during the inoculation.

By calculating the doubling time for the cells, cell growth can be estimated and compared between different sized systems. Doubling time was calculated using the formula: Nt = N02t/d where: Nt = cell number at time t, N0 = initial cell number, t = time, d = doubling time. Doubling time for the HEK293 cells in our earlier flask experiments was 28.8 hours (data not shown). In the iCELLis^{®} Nano, the doubling time was 29.6 hours and in the iCELLis^{®} 500 it was 29.0 hours. Indicating cell growth appears to be comparable between flask system and iCELLis^{®}. This demonstrates the practicality of the iCELLis^{®} technology for cell growth.

**Table 1. Cell distribution (cells/cm²) based on nuclei count in iCELLis^{®} Nano and iCELLis^{®} 500 after five days cultivation. 13 000 cells/cm² were loaded into iCELLis^{®} Nano and 6000 cells/cm² into iCELLis^{®} 500**

| **Run ID** | **Bottum** | **Middle Top** | |
|---|---|---|---|
| iCELLis^{®} Nano (4m²) | 325 200 | 112000 | 60 600 |
| Average | 172 600 | | |
| iCELLis^{®} 500 (500m²) | 136 800 | 85 900 | 88 200 |
| Average | 103 600 | | |

Suspension cells were also split into normal 175 cm2 cell culture flasks at 4,5 × 10⁶ cells per flask. Two hours after the inoculation, cell attachment onto the bottom of the cell flasks was seen under the microscope. On day 5, the cell confluence was about 70% and there were totally 30.3 × 10⁶ cells in one flask. This proved that suspension adapted HEK293 cells expanded using a suspension process were also able to be attached onto the bottom of the cell culture flask, and grow there.

### 4. Inoculation

Inoculation of iCELLis^{™} nano Bioreactor after expansion of cells either in spinner flasks or in roller bottles were studied in detail. During the first experiment, after cell counting, 5.2×10⁸ suspension cells (equivalent to 1.3×10⁴ cells/cm2) were centrifuged (209 g, 5 min, 20 °C) and resuspended to 200 ml DMEM+ FBS growth medium. Cell grow was monitored by counting cells (nuclei) from the top carriers (Figure 2a).

We studied the possibility of missing out the centrifugation step and inoculated cells directly from suspension (in suspension medium) to the bioreactor containing adherent cell culture medium. First, a flask experiment was performed. Suspension adapted HEK293 cells were cultured in adherent flasks using different ratios of DMEM and ExCell medium (100, 95, 90, 85, 80, 70 or 60% DMEM, rest of being ExCell). In the second experiment the cells were also infected to see the effect of ExCell residues for the productivity. Low Excell concentration did not have as major an effect on cell grow as adherence. When the ExCell concentration was increasing, cells started to look more rounded and were not attached very firmly on the bottom of the flask. Also it was observed that residual 5% Excell in DMEM has only a minor effect on productivity; the decrease was 7% (data not shown). 10 % ExCell decreases the total yield already with 19%, and 15% FBS decreases 63%, respectively. In the flask experiment no perfusion could be performed and tested like it will be done in the bioreactor.

We tested the inoculation of suspension cells in 100-200 ml of ExCell medium into the iCELLis Nano bioreactor containing 600 ml pre-equilibrated adherent growth medium with or without FBS. If FBS was not present during the inoculation, it was added 10min -2 h post inoculation. Stirring speed was set to 1050 rpm (2 cm/sec) for the first 2 h, and then reduced to 755 rpm (1 cm/sec). After speed reduction, cell attachment was followed by taking a small medium sample and counting cells. The number of attached cells was determined from the carriers by counting the nuclei. Based on this experiment, cells attached onto carriers even if they had been earlier adapted to grow in suspension. We were also looking if FBS had some effect on cell distribution but based on the cell number on the top carriers, it was concluded that the presence of FBS during the inoculation did not have any effect on cell distribution and cell attachment onto carriers (figure 2a).

Not only HEK293 but also 293T cells were expanded in suspension using spinner flasks and EXCell medium and 7000 cells/cm² were inoculated into the iCELLis Nano 4m² bioreactor. 293T cells attached on the carriers and the growth was monitored from the top carriers and also following the glucose consumption and lactate accumulation (figure 2b). 293T cells were growing well in the iCELLis bioreactor as adherence after suspension expansion.

As a conclusion, two different cell lines were tested for the suspension expansion prior to adherent manufacturing. It was noticed that the inoculation method is practical, directly from the disposable suspension bioreactor into a iCELLis bioreactor without any further process steps. This is a major advantage and makes this innovation very cost-effective and practical. All the large scale iCELLis bioreactor runs were inoculated directly from Cultibag bioreactor (see also 3. suspension expansion)

### 6. Infection/transfection/transduction conditions

After growing the suspension expanded HEK293 cells on adherent mode for four to five days, cells were infected (transduced) by adenovirus serotype 5 viral vector (Ad5). In our experiment, MOI varied between 50 and 400. To iCELLis Nano bioreactor the required volume of Ad5 was pipetted either into 800 ml pre-warmed growth medium and cells were infected by full medium exchange, or virus was added in a small volume directly into the bioreactor without any medium removal. After the infection, the virus-containing medium was replaced with fresh medium. Additionally virus medium was not removed, instead the perfusion was started to support the cell growth. During the perfusion, remaining virus was finally removed into waste.

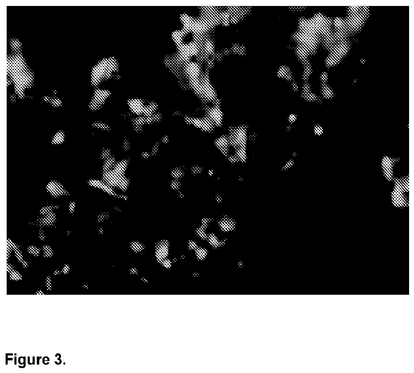

Transfection example was performed by calcium phosphate-mediated transfection to produce AAV. Suspension-expanded 293T cells were inoculated into the bioreactor and cell mass was further expanded in a iCELLis bioreactor for four days. The cell grow curve is shown in figure 2b. Cells were transfected with two plasmids and the calcium phosphate method. Plasmid transfection method of suspension expanded cells was monitored 24h post transfection by fluorescent microscope. The transfection had been efficient (figure 3).

Commercial adherent bioreactors are typically provided with an automatic pH control to maintain the culture medium at a constant pH, automatically adding a basic solution (e.g., a sodium bicarbonate solution) if the culture medium pH falls. We found that if the automatic pH control in the iCELLis^{™} bioreactor is left operational, then the bioreactor will add base solution into the bioreactor, which will cause the formation of a precipitate in the bioreactor. That precipitate, which we believe is a DNA-salt precipitate, is undesirable because it clogs the bioreactor and impedes productivity. We found that by disabling the automatic pH control after transfection and allowing the pH of the culture medium to fall naturally, the resulting slightly-acidic culture medium prevents precipitate formation and thus increases yield.

### 7. The Product and productivity

In this proof-of-concept experiments adenovirus type 5 expressing human interferon α 2b was used as an examples (Dinney *et al.* 2013). Concept examples are not limited to this product only. Plasmid transfection was demonstrated using Adeno-associated virus serotype 2 expressing green fluorescent protein as a marker gene.

To study the virus production after suspension expansion, three iCELLis^{®} Nanos were operated in order to show the proof-of-concept of suspension expansion step prior inoculation. HEK293 cells were cultured in serum-free suspension conditions in two ways, firstly in roller bottles and alternatively in a BIOSTAT^{®} CultiBag RM perfusion system. For the Cultibag, first suspension adapted cells (500 × 10⁶ cells in 200 ml) from the roller bottles was transferred into CultiBag RM perfusion system, where 800 ml of growth medium Ex-Cell medium containing 6 mM L-glutamine and 50 µg/ml penicillin/streptomycin had already been equilibrated. Thus a starting cell concentration of 0.5 × 10⁶ cells/mL in 1L working volume was achieved. The cells were expanded for four days up to 2 - 4 × 10⁶ cells/ml in 1L working volume. Perfusion was started when cell density reached ≥ 1.0 × 10⁶ cells/ml and continued throughout the expansion. During Cultibag ^{RM} perfusion fresh growth medium was introduced to CultiBag ^{RM} at the same rate as waste medium was removed. Perfusion rate was one working volume (1L) per 24 hours. 239T cells were cultured in serum-free conditions in spinner flasks. The cells from the suspension expansion were transferred into the iCELLis^{®} Nano bioreactor - those HEK293 cells from the roller bottles to the 0.8m2 (Run 1) and those from the BIOSTAT^{®} Cultibag ^{RM} to the 4m2 (Run 2) iCELLis^{®} Nano. 293T cells were transferred also to 4m² iCELLis Nano^{®}. Most of the cells were initially entrapped into the fixed bed in less than 30 min, after which all the cells were attached onto the carriers and continued growth in adherent mode. The growth of the adherent HEK293 and 293Tcells expanded in suspension was monitored by calculating nuclei (cells) from the top carriers each day during the culture. Both cell lines attached onto carriers and exponential growth was observed (figure 2).

HEK293 cells were infected by adenovirus and their productivity was 5.6 × 10⁶ (total of 4.4 × 10¹³ vp) and 2.6. × 10⁶ vp/cm² (total of 1.0 × 10¹⁴ vp), respectively (Table 1). Ad5 titering was performed by HPLC based viral particle titering method. AAV was produced in 293T cells by calcium phosphate mediated plasmid transfection and productivity was 1.1 × 10⁹ VG/cm² , (total of 4.5 × 10¹³ VG, viral genome analyzed by qPCR-based titering method). Suspension expansion did not have major effect on the productivity compared to the productivity if cells were expanded in adherent mode.

Process was scaled up and for the first time iCELLis^{®}500 100m² was tested for the cell growth after the suspension followed by adenovirus infection and production. In this experiment HEK293 cells in suspension were seeded in a density of 0.5 × 10⁶/ml and expanded into density of 1.9 × 10⁶ cells/ml, using a BIOSTAT^{®} CultiBag RM perfusion system. At the time of inoculation, 1×10¹⁰ cells were inoculated directly into the iCELLis^{®}500 100m² bioreactor containing equilibrated growth medium. During the experiment, glucose, lactate and pH were measured off-line daily. Perfusion was started later to support the cell growth with fresh medium. The cells were infected and perfusion was continued to support the cell viability and productivity. Glucose consumption and lactate accumulation were comparable for Nano experiments. The harvest yield was 5.3 × 10¹⁵ vp and in a rinse there were still 8.4 ×10¹⁴ vp found representing 14% of the total yield. The total virus yield from bioreactor was 6.1×10¹⁵ vp and the productivity 6.1×10⁹ vp/cm2 (Table 1, Run 3).

**Table 2. Comparing the productivity of adenovirus in iCELLis Nano^{®} after different cell expansion methods.**

| **System** | **Run nro** | **Explantion** | **Yield (total vp)** | **Area (cm²)** | **Productivity (vp/cm²)** |
|---|---|---|---|---|---|
| Flask | | Control flasks | 8.1 × 10¹¹ | 175 | 4.6 × 10⁹ |
| iCELLis^{®} Nano 0.8m² | Run 1 | Suspension expansion in roller bottles | 4.4 × 10¹³ | 7776* | 5.6 × 10⁹ |
| iCELLis^{®} Nano 4m² | Run2 | Suspension expansion in BIOSTAT^{®} CultiBag RM | 1.0 × 10¹⁴ | 40 000 | 2.6 × 10⁹ |
| iCELLis^{®}500 100m² | Run 3 | Upscaling the process. Suspension expansion in BIOSTAT^{®} CultiBag RM | 6.1 × 10¹⁵ | 1000 000 | 6.1 × 10⁹ |

It is notable that iCELLis^{®}500 is providing even more scalability than shown in an example. The productivity was tested in 100m² bioreactor whereas the maximal area of the bioreactor is 500m² which could theoretically produce five times more viral particles which is in this case 2.65 × 10¹⁶ vp. As explained previously, particular product was a low producer. With this product we saw that the productivity achieved in flasks was remained the same even in large scale.

Interesting calculation can be performed by using one of our "high producers" as an example. We have seen that the productivity in flasks was 2.23 × 10¹⁰ vp/cm². If this number is converted to 100m², it would reach the total of 2.23 × 10¹⁶ vp harvest yield and further into 1.12 × 10¹⁷ harvest yield in 500m². Other words, we have been able to develop a method that can meet the requirements for commercial scale and solve the current problem in scaling up the manufacturing process.

### 8. Harvest

Different harvest methods were tested. The harvest can be performed e.g. at 48 hrs p.i. before the lytic adenovirus had been released from the producer cells into the cell culture medium. Medium sample, taken at 48h p.i. for HPLC analysis showed that the viral particle number was below the detection limit, proving that significant virus had not yet occurred and viruses were still inside the producer cells. The iCELLis^{®} bioreactor fixed-bed proved not very practical for detaching cells by Trypsin or derivatives, nor collecting them. It was thus decide to test the release of the virus into the cell culture medium by chemical lysis buffer (Tween20). To understand the efficacy of in-situ lysis/harvest, 22 carriers were lyzed in a water bath for 2 h at 37 °C, with 1 % of lysis buffer in the growth medium, and manual shaking every 10-15 min. The rest of the iCELLis^{®} carriers were tested in-situ within the bioreactor for 2 h at 37°C, with 1 % Lysis buffer in the growth medium and this was continuously circulated through the fixed carrier bed. The virus yield from the iCELLis^{®} bioreactor was a total of 7.3×10¹³ vp and productivity was 9.9×10⁹ vp/cm2. The amount of viral particles released from the carriers when lysed in water bath was lower than when lysis was done inside the iCELLis^{®}. As a further control, cells from the control flasks were also trypsinized and harvested, followed by virus release with the traditional freeze-and-thaw method. This showed a productivity of 4.0 × 10⁹ vp/cm2. These results proved that the release of the virus from the cells lysed inside the iCELLis^{®} fixed-bed had been efficient. The same lysis method for adenovirus from HEK293 cells was tested also for releasing AAV from 293T cells. Release of AAV was efficient reaching to total yield of total of 4.5 × 10¹³ VG.

For further recovery of the virus, rinsing step after the lysis and harvest was tested, which is very unique method and not applicable for many type of biroectors. When bioreactor was rinsed with normal cell culture medium such as DMEM, less virus particles were recovered than when the bioreactor was rinsed with buffer containing lysis detergent.

### 8.5 Harvest Using Hypotonic Solution

To increase yield, one may lyse producer cells and harvest the product from the lysed cells. To lyse cells, one can use a hypotonic solution. To test this, we performed a hypotonic cell lysis experiment in an iCELLis^{™} nano bioreactor. In this method, cells were exposed to a solution with a much lower solute concentration. The difference in solute concentration creates an osmotic pressure gradient. In an attempt to balance the concentrations inside and outside the cells, water will flow into the cells and can cause them to swell and burst. In this experiment, the infected cells were lysed by incubating them in water for irrigation.

Suspension cultured HEK293 cells were inoculated into a 4 m² iCELLis nano bioreactor and expanded for approx. 120 hours using DMEM - 10 % - 1 % L-Glutamine - 1 % Pen-Step. After expansion in the bioreactor, cells were infected with pre-amplified DVL WVSS 01 (rAd-IFN) using MOI 200. Approx. 24 hours post-infection, serum free medium was started to perfuse. Approx. 56 hours post-infection, harvest was started by transferring the spent medium from the bioreactor into a separate container. 700 ml of water for irrigation was transferred into the bioreactor and stirred at 100 % for 20 - 30 min w/o heating. Harvest material was then transferred into a separate bag. To rinse the fixed bed, 600 ml of water for irrigation was transferred into the bioreactor and stirred at 100 % for 5-10 min w/o heating. Rinse material was then transferred into the bag and mixed with the harvest material. Samples were taken from each step and adenoviral particle concentration was analysed by AEX-HPLC (Table I).

**Table I. Viral particle yield from hypotonic cell lysis experiment.**

| **Sampling step** | **Volume (ml) (approx)** | **Virus concentration (vp/ml) (AEX**_**15_032)** | **Total virus yield (vp)** |
|---|---|---|---|
| Medium | 900 | not detected | - |
| WFI harvest (weighted) | 693 | 1,78E+10 | 1,23E+13 |
| WFI rinse | 600 | 2,59E+10 | 1,55E+13 |
| Pooled harvest (weighted) | 1338 | 2,88E+10 | **3,85E+13** |

### 9. DNA digestion and prevention of precipitation/aggregation

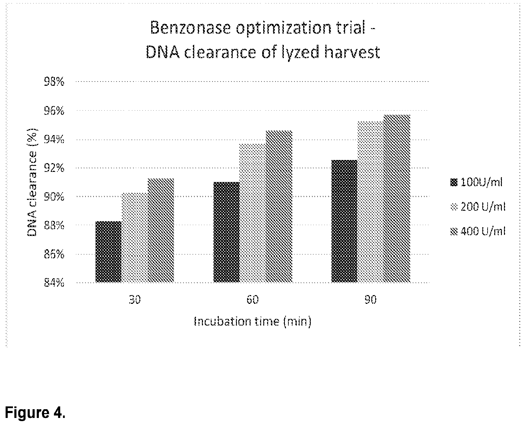

DNA removal from the final product is required by the authorities. The removal of the DNA is started by digesting DNA enzymatically. When Benzonase^{™} treatment was tested, the viral containing harvest was transferred into separate vessels and treated with increasing concentration of Benzonase^{™} (100-400u/ML). The effect of incubation time was evaluated too (30- 90 min.) This experiment was performed by incubating the harvested material in 37°C. When Benzonase^{™} treatment was done in separate vessels, 96% genomic DNA removal was achieved using 400U/ml and 90 min incubation time. When the product was further downstream processed, no viral aggregation or precipitation was observed after the harvest or during the downstream processing. These results showed that one step digesting of cellular nucleic acid is efficient when performed in a separate vessel than the inside the bioreactor. The product was harvested from the bioreactor when most of the cell debris was still remained in a fixed bed (Figure 4).

Alternatively, the DNA digestion was tested inside the bioreactor when Benzonase^{™} treatment was performed before and after the producer cell chemical lysis followed by addition of high salt containing buffer. Formation of precipitation was studied by measuring the turbidity (FNU) of the suspension. Based on our findings, digestion of the DNA, addition of lysis detergent (e.g., Tween^{™} or Triton-X^{™}) and high salt prevents the formation of precipitation. High concentration salt solution added to reach 1000 mM NaCl. Final harvest sample contains material which was treated with pre-Benzonase^{™}, Tween-20 based lysis, Benzonase^{™} and diluted in a buffer containing 1M NaCal, 50 mM Hepes, 1% m/V Tween 20, 1% glycerol when no increase of turbidity was detected (Figure 5).

### Modification

No modification of cells or long adaptation techniques were used as completely contra to expectation. Non adherence cultures provided by the FVT system were able to adhere to the iCELLis nano carriers.

### Results and Discussion

This Example demonstrates the expansion of the non-anchorage-dependent suspension-adapted HEK 293 cells in a suspension bioreactor (which can be, for example, the Cultibag by Sartorius or a corresponding wave bioreactor, or indeed any kind of stirred tank bioreactor or shaker flask system for suspension adapted cells) and their inoculation into an iCELLis bioreactor, to be cultured in an adherence system.

All published reports introduce two dimensional adherent growth using, for example, adherent roller bottles, cell factories or cell stacks as the only options for adherent cell expansion. Gelbras et al. have been studying the iCELLis bioreactor, its function, and cell growth within it. They note that when adherent cells are inoculated into the bioreactor, cells need to first be detached from the bottom of the adherent flask/bottle (in which they are expanded) using e.g. trypsin (or analogous). Cells are suspended into cell growth medium and inoculated into the bioreactor. These cells are still typically anchorage-dependent cells. As studied by Gelbras et al., forming first cell suspension for inoculation, they precisely state that the cell growth and the cell death are assumed to be negligible only during the adherence phase. The only tip from the manufacturer's specialist was to study the absolute minimum cell density and so as to minimise the number of cells required for inoculation. This all states that no one has previously considered using any kind of suspension cell systems for expanding cells to be inoculated into any size of adherent type bioreactor, as the suspension process adapted cells have been modified not to adhere.

Given our disclosure, the artisan can readily make variations to achieve the benefit of our research in different specific applications. For example, while we exemplify lysing producer cells which have been cultured using adherent cell culture, the artisan could readily use the same technique to lyse producer cells which have been cultured in suspension mode. We thus intend the coverage of our patent to be defined not by our Examples discussed above, but by the legal claims recited below. In the appended claims, the singular ("a," "one") encompasses the plural. We use the term "transfection" to broadly encompass introducing foreign, expressible nucleic acid sequences into a host cell; this includes e.g., transformation, transduction and infection with a viral vector. Method steps may be performed in any order, except where expressly noted in the legal claim.

### References

Dinney, C. P., M. B. Fisher, N. Navai, M. A. O'Donnell, D. Cutler et al. 2013 Phase I trial of intravesical recombinant adenovirus mediated interferon-alpha2b formulated in Syn3 for Bacillus Calmette-Guerin failures in nonmuscle invasive bladder cancer. J. Urol. 190: 850-856.
Dormond, E., M. Perrier, and A. Kamen, 2009 From the first to the third generation adenoviral vector: what parameters are governing the production yield? Biotechnol.Adv. 27: 133-144.
Iyer, P., J. M. Ostrove, and D. Vacante, 1999 Comparison of manufacturing techniques for adenovirus production. Cytotechnology 30: 169-172.
Kamen, A., and O. Henry, 2004 Development and optimization of an adenovirus production process. J.Gene Med. 6 Suppl 1: S184-S192.
Lennaertz A., Knowles S., Drugmand JC. & Castillo J. Viral vector production in the integrity® iCELLis® single-use fixed-bed bioreactor, from bench-scale to industrial scale. 7 (Suppl 6), 59-60. 2013. BMC Proceedings.
   Ref Type: Generic
Meuwly, F., P. A. Ruffieux, A. Kadouri, and S. U. von, 2007 Packed-bed bioreactors for mammalian cell culture: bioprocess and biomedical applications. Biotechnol.Adv. 25: 45-56.
Salmon, F., K. Grosios, and H. Petry, 2014 Safety profile of recombinant adeno-associated viral vectors: focus on alipogene tiparvovec (Glybera(R)). Expert.Rev.Clin.Pharmacol. 7: 53-65.
Segura, M. M., A. Garnier, Y. Durocher, H. Coelho, and A. Kamen, 2007 Production of lentiviral vectors by large-scale transient transfection of suspension cultures and affinity chromatography purification. Biotechnol.Bioeng. 98: 789-799.
Stacey, G. N., and O. W. Merten, 2011 Host cells and cell banking. Methods Mol.Biol. 737: 45-88.
Vellinga, J., J. P. Smith, A. Lipiec, D. Majhen, A. Lemckert et al. 2014 Challenges in manufacturing adenoviral vectors for global vaccine product deployment. Hum.Gene Ther. 25: 318-327.
Wu, S. C., G. Y. Huang, and J. H. Liu, 2002 Production of retrovirus and adenovirus vectors for gene therapy: a comparative study using microcarrier and stationary cell culture. Biotechnol.Prog. 18: 617-622.

## Claims

1. A method for the production of a recombinant biological product, comprising:
(a) obtaining cells that have been expanded in suspension mode; and
(c) inoculating the cells into a bioreactor having a packed-fiber carrier providing a surface area for adherent cell culture; and then
(d) exposing the cells to a factor promoting adherence selected from the group consisting of: fetal bovine serum, collagen, fibronectin, laminin, calcium ions, proteoglycans and ECM polysaccharides, in an amount effective to promote adherence, whereby the cells adhere to said packed-fiber carrier in said bioreactor.

2. The method according to claim 1, further comprising:
(b) before said inoculating step, expanding the cells in non-adherent mode.

3. The method of claim 1, further comprising:
(e) after the cells adhere to the carrier, introducing a transgene into the cells.

4. The method of claim 3, wherein the transgene is introduced into the cells after said inoculating step.

5. The method according to any one of claims 1-4, further comprising:
(f) harvesting the recombinant biological product.

6. The method according to any one of claims 1-5, wherein the cells that have been expanded in suspension mode are selected from the group consisting of HEK293, 293T, or any HEK293 related cell line HT-1080, HepG2, A549, CHO, BHK, Sf9, Sf21, NCL, UR, N52.E6, BTI-Tn 5 B 1-4, COS, NIH/3T3, Vero, NSO, NC5Tl 1, PerC6 or Her cells;
optionally wherein the cells that have been expanded in suspension mode are selected from HEK293 and 293T cells.

7. The method according to any one of claims 1-6, wherein the recombinant biological product is a
(i) viral vector,
(ii) viral-like particle,
(iii) virus,
(iv) recombinant protein,
(v) immunoglobulin, or
(vi) viral-based vaccine.

8. The method according to claim 7(i), wherein the viral vector comprises a therapeutic transgene.

9. Use of suspension-adapted cells in a method for the production of a recombinant biological product; wherein the cells have been expanded in a suspension cell culture; and wherein the method comprises inoculating the expanded cells obtained from the suspension culture into a bioreactor having a packed-fiber carrier, and then exposing the cells to a factor promoting adherence selected from the group consisting of: fetal bovine serum, collagen, fibronectin, laminin, calcium ions, proteoglycans and ECM polysaccharides, in an amount effective to promote adherence, whereby after inoculating the cells, the cells adhere to the packed-fiber carrier in the bioreactor and the expanded cells are cultured in adherent mode to produce the recombinant biological product.

## Patentansprüche

1. Ein Verfahren zur Herstellung eines rekombinanten biologischen Produkts, umfassend:
(a) Erhalten von Zellen, die im Suspensionsmodus expandiert worden sind; und
(c) Inokulieren der Zellen in einen Bioreaktor mit einem gepackten Faserträger, der eine Oberfläche für eine adhärente Zellkultur bereitstellt; und dann
(d) Aussetzen der Zellen einem Faktor, der die Adhärenz fördert, wobei der Faktor ausgewählt ist aus der Gruppe bestehend aus: fötalem Rinderserum, Kollagen, Fibronektin, Laminin, Calciumionen, Proteoglykanen und ECM-Polysacchariden, in einer Menge, die wirksam ist, um die Adhärenz zu fördern, wodurch die Zellen an dem gepackten Faserträger in dem Bioreaktor anhaften.

2. Das Verfahren nach Anspruch 1, ferner umfassend:
(b) vor dem Inokulationsschritt das Expandieren der Zellen im nicht-adhärenten Modus.

3. Das Verfahren nach Anspruch 1, das ferner umfasst:
(e) nach dem Anhaften der Zellen an den Träger das Einbringen eines Transgens in die Zellen.

4. Das Verfahren nach Anspruch 3, wobei das Transgen in die Zellen nach dem Inokulationsschritt eingebracht wird.

5. Das Verfahren nach einem der Ansprüche 1-4, ferner umfassend:
(f) Ernten des rekombinanten biologischen Produkts.

6. Das Verfahren nach einem der Ansprüche 1-5, wobei die Zellen, die im Suspensionsmodus expandiert wurden, ausgewählt sind aus der Gruppe bestehend aus HEK293, 293T oder einer HEK293-ähnlichenre Zelllinie HT-1080, HepG2, A549, CHO, BHK, Sf9, Sf21, NCL, UR, N52.E6, BTI-Tn 5 B 1-4, COS, NIH/3T3, Vero, NSO, NC5Tl 1, PerC6 oder Her-Zellen;
optional wobei die Zellen, die im Suspensionsmodus expandiert wurden, aus HEK293- und 293T-Zellen ausgewählt sind.

7. Das Verfahren nach einem der Ansprüche 1 bis 6, wobei es sich bei dem rekombinanten biologischen Produkt um
(i) einen viralen Vektor,
(ii) einen virenähnlichen Partikel,
(iii) einen Virus,
(iv) ein rekombinantes Protein,
(v) ein Immunglobulin, oder
(vi) einen Impfstoff auf viraler Basis
handelt.

8. Das Verfahren nach Anspruch 7(i), wobei der virale Vektor ein therapeutisches Transgen umfasst.

9. Verwendung von suspensionsadaptierten Zellen in einem Verfahren zur Herstellung eines rekombinanten biologischen Produkts; wobei die Zellen in einer Suspensionszellkultur expandiert wurden; und wobei das Verfahren das Inokulieren der expandierten Zellen, die aus der Suspensionskultur erhalten wurden, in einen Bioreaktor mit einem gepackten Faserträger und das anschließende Aussetzen der Zellen einem Faktor, der die Adhärenz fördert, umfasst, wobei der Faktor ausgewählt ist aus der Gruppe bestehend aus: fötalem Rinderserum, Kollagen, Fibronektin, Laminin, Kalzium-Ionen, Proteoglykanen und ECM-Polysacchariden, in einer Menge, die wirksam ist, um die Adhärenz zu fördern, wobei nach dem Inokulieren der Zellen die Zellen an dem gepackten Faserträger in dem Bioreaktor anhaften und die expandierten Zellen im adhärenten Modus kultiviert werden, um das rekombinante biologische Produkt herzustellen.

## Revendications

1. Méthode pour la production d'un produit biologique recombinant, comprenant :
(a) l'obtention de cellules qui ont été expansées en mode suspension ; et
(c) l'inoculation des cellules dans un bioréacteur ayant un support en fibres compactées fournissant une surface pour la culture de cellules adhérentes ; et puis
(d) l'exposition des cellules à un facteur favorisant l'adhérence choisi dans le groupe constitué de : sérum foetal de bovin, collagène, fibronectine, laminine, ions calcium, protéoglycanes et polysaccharides de la matrice extracellulaire, en une quantité efficace pour favoriser l'adhérence, moyennant quoi les cellules adhèrent audit support en fibres compactées dans ledit bioréacteur.

2. Méthode selon la revendication 1, comprenant en outre :
(b) avant ladite étape d'inoculation, l'expansion des cellules en mode non adhérent.

3. Méthode selon la revendication 1, comprenant en outre :
(e) après que les cellules ont adhéré au support, l'introduction d'un transgène dans les cellules.

4. Méthode selon la revendication 3, dans laquelle le transgène est introduit dans les cellules après ladite étape d'inoculation.

5. Méthode selon l'une quelconque des revendications 1 à 4, comprenant en outre :
(f) la récolte du produit biologique recombinant.

6. Méthode selon l'une quelconque des revendications 1 à 5, dans laquelle les cellules qui ont été expansées en mode suspension sont choisies dans le groupe constitué des cellules HEK293, 293T, ou l'une quelconque des cellules de lignée cellulaire HT-1080 apparentée à HEK293, HepG2, A549, CHO, BHK, Sf9, Sf21, NCL, UR, N52.E6, BTI-Tn 5 B 1-4, COS, NIH/3T3, Véro, NSO, NC5Tl 1, PerC6 ou Her ;
éventuellement dans laquelle les cellules qui ont été expansées en mode suspension sont choisies parmi les cellules HEK293 et 293T.

7. Méthode selon l'une quelconque des revendications 1 à 6, dans laquelle le produit biologique recombinant est
(i) un vecteur viral,
(ii) une particule de type viral,
(iii) un virus,
(iv) une protéine recombinante,
(v) une immunoglobuline, ou
(vi) un vaccin à base virale.

8. Méthode selon la revendication 7(i), dans laquelle le vecteur viral comprend un transgène thérapeutique.

9. Utilisation de cellules adaptées à la suspension dans une méthode de production d'un produit biologique recombinant ; dans laquelle les cellules ont été expansées dans une culture cellulaire en suspension ; et dans laquelle la méthode comprend l'inoculation des cellules expansées obtenues à partir de la culture en suspension dans un bioréacteur ayant un support en fibres compactées, et puis l'exposition des cellules à un facteur favorisant l'adhérence choisi dans le groupe constitué de : sérum foetal de bovin, collagène, fibronectine, laminine, ions calcium, protéoglycanes et polysaccharides de la matrice extracellulaire, en une quantité efficace pour favoriser l'adhérence, moyennant quoi après inoculation des cellules, les cellules adhèrent audit support en fibres compactées dans le bioréacteur et les cellules expansées sont cultivées en mode adhérent pour produire le produit biologique recombinant.
